# EUROPEAN PATENT SPECIFICATION

(11) **EP 1 550 469 B1**
(45) Date of publication and mention of the grant of the patent: **02.11.2006**
(21) Application number: 03258255.3
(22) Date of filing: 31.12.2003
(51) Int. Cl.: A61L 15/60, A61L 15/62

(54) **Stable biodegradable, water absorbing gamma-polyglutamic acid hydrogel**
Stabiler, biologisch abbaubares, wasserabsorbierendes Gamma-Polyglutaminsäure- Hydrogel
Hydrogel à base d'acide gamma polyglutamique, stable, biodégradable, absorbant l'eau

(30) Priority: 19.12.2003 JP 2003423533
(43) Date of publication of application: 06.07.2005
(73) Proprietor: Tung Hai Biotechnology Corporation, Taichung Hsien (TW)
(72) Inventor: Ho, Guan-Huei, Mississauga, Ontario L5N 7Y6 (CA); Yang, Tou-Hsiung, Taichung Hsien, Taiwan (TW); Yang, Kung-Hsiang, Taichung Hsien, Taiwan (TW)
(74) Representative: Sexton, Jane Helen

(56) References cited:
- WO-A-20/04007593
- PATENT ABSTRACTS OF JAPAN vol. 2000, no. 03, 30 March 2000 (2000-03-30) & JP 11 343339 A (HARA TOSHIO; IDEMITSU KOSAN CO LTD), 14 December 1999 (1999-12-14)
- PATENT ABSTRACTS OF JAPAN vol. 1996, no. 03, 29 March 1996 (1996-03-29) & JP 07 310021 A (NIPPON SHOKUBAI CO LTD), 28 November 1995 (1995-11-28)
- PATENT ABSTRACTS OF JAPAN vol. 1997, no. 05, 30 May 1997 (1997-05-30) & JP 09 020614 A (AJINOMOTO CO INC), 21 January 1997 (1997-01-21)
- PATENT ABSTRACTS OF JAPAN vol. 2003, no. 02, 5 February 2003 (2003-02-05) & JP 2002 305975 A (SAKATA KYOKO), 22 October 2002 (2002-10-22)
- PATENT ABSTRACTS OF JAPAN vol. 1995, no. 02, 31 March 1995 (1995-03-31) & JP 06 322358 A (AGENCY OF IND SCIENCE & TECHNOL), 22 November 1994 (1994-11-22)
- PATENT ABSTRACTS OF JAPAN vol. 1998, no. 03, 27 February 1998 (1998-02-27) & JP 09 296039 A (EIWEISS KK), 18 November 1997 (1997-11-18)
- PATENT ABSTRACTS OF JAPAN vol. 2003, no. 12, 5 December 2003 (2003-12-05) & JP 2004 210700 A (ICHIMARU PHARCOS CO LTD), 29 July 2004 (2004-07-29)

## Description

### Field of the Invention

The present invention relates to a stable biodegradable, high water absorbable γ-polyglutamic acid (γ-PGA) hydrogel by 3-dimensional cross-linking, and its preparation method and uses.

### Background of the Invention

In recent years, water absorbable hydrogels have been used not only as the materials for paper diapers and tampons, but also as liquid absorbents for use in medical care, construction, civil engineering, building, etc. Moreover, the water absorbable hydrogels can also be used as texture enhancers, fresh-keeping agents for foods, important basal material for greening engineering in the fields of horticulture and other agricultural applications.

Conventional methods for preparing water absorbable hydrogels use starches and celluloses cross-linked with acrylnitrile to form acrylate-based water absorbable hydrogels. Although such acrylate-based hydrogels are cheap, they can be partially decomposed by microorganisms in the soil and encounter difficulties in waste treatment and problems of toxicity toward human bodies. It is believed that imparting water absorbable hydrogels with good biodegradability will resolve the problem regarding the waste treatment. Therefore, there is a great demand for biodegradable, water absorbable hydrogels in view of the increasing environmental concerns.

In order to overcome the aforementioned problem, conventional techniques involve using biodegradable starch-based, hyaluronic acid-based, or polyamino acid-based, or materials as the starting materials for the preparation of biodegradable, water absorbable hydrogels. The methods for the preparation of polyamino acid-based, cross-linked products have been disclosed in the prior art, such as JP 6-322358, JP 7-224163, JP 7-309943, JP 7-300563, JP 10-298282, and JP 11-343339. For example, JP 6-322358 indicates that a solution of γ-PGA can be cross-linked via an electronic polymerization mechanism by using strong gamma-irradiation, so as to form γ-PGA based, cross-linked product. However, the equipments for producing γ-PGA based, cross-linked products through irradiation is very complicated and restricted, such that the production procedure is difficult and inconvenient. JP 11-343339 discloses another method for preparing cross-linked γ-PGA product, comprising isolating a high concentration γ-PGA from a culture broth, and using the isolated γ-PGA as the starting material for the cross-linking reaction with a di-epoxy compound to obtain a biodegradable, water absorbable hydrogel. Nonetheless, such method not only has the drawback associated with the requirement of a high concentration of γ-PGA, obtained through the procedures including a cell separation and extraction of γ-PGA from a microbial culture broth through a refining step, but also requires particular operational equipments to improve the solubility of γ-PGA and the di-epoxy compound in a solvent. Moreover, the above method uses γ-laser irradiation to complete the cross-linking reaction between γ-PGA and the di-epoxy compound. Obviously, the preparation technology disclosed in JP 11-343339 also causes the problems regarding an increase in cost and inconvenience in preparation procedure.

Moreover, JP 5-301904 discloses polysaccharides produced from Alcaligenes letus B16. US 4,772,419 also discloses a method for the preparation of cross-linked polysaccharide products.

Conventional methods for manufacturing cross-linked γ-PGA products require complicated processing procedures, such as the control and operation of complicated irradiation equipments and the separation and refining steps. Also, γ-PGA hydrogel products obtained by known technology are relatively unstable and easily decomposed in a few days (3 to 5 days) after fully swelling in water at room temperature. Surprisingly, the inventors of the present application have found that good biodegradable, high water absorbable γ-PGA hydrogels having up to 5,000 times water absorption capacity, improved firmness, and long-term stability after full swelling in water or an aqueous medium can be directly, simply, and successfully prepared.

### Brief Description of the Invention

The present invention relates to a method for the production of a biodegradable water absorbable γ-polyglutamic acid (γ-PGA) hydrogel comprising directly cross-linking.
(A) a γ-polyglutamic acid (γ-PGA), a γ-polyglutamate, or a mixture thereof, and optionally a polysaccharide containing a carboxylic and/or carboxylate group, an amino acid, or a mixture thereof; and/or
(B) a microbial culture broth containing a γ-polyglutamic (γ-PGA), a γ-polyglutamate or a mixture thereof, and optionally a polysaccharide containing a carboxylic and/or carboxylate group, an amino acid, or a mixture thereof,
with a cross-linker comprising a polyglycidyl ether,
wherein each of the functional groups of the polyglycidyl ether can react with a carboxylic group (-COOH), carboxylate group (-COO⁻), aldehyde group (-CHO), hydroxyl (-OH), carbonyl group (-CO), sulfone group (-SO₂), amino group (-NH₂) or nitro group (-NO₂), or a mixture thereof.

The present invention further relates to a biodegradable, water absorbable (γ-PGA) hydrogel prepared by the above method. The inventive γ-PGA hydrogel exhibits good biodegradability, up to 5,000 times water absorption capacity, and improved firmness, and long-term stability after fully swelling in water or an aqueous medium.

### Detailed description of the Intention

In the method of the present invention, (A) a γ-polyglutamic acid (γ-PGA), a γ-polyglutamate, or a mixture thereof, and optionally a polysaccharide containing a carboxylic and/or carboxylate group, an amino acid, or a mixture thereof; and/or (B) a microbial culture broth containing a γ-polyglutamic acid (γ-PGA), a γ-polyglutamate, or a mixture thereof, and optionally a polysaccharide containing a carboxylic and/or carboxylate group, an amino acid, or a mixture thereof directly react with a cross-linker comprising a polyglycidyl ether. Preferably, γ-PGA with a molecular weight of more than 100,000 Daltons is used. The polysaccheride may be selected from, but is not limited to a mixture of glucose, fructose, galactose, and glucuronic acid, and a mixture of rhamnose, glucose, galactose, and glucuronic acid, and a polycarboxylic acid in which hyaluronic acid as the principal component. As for the amino acid, it can be selected from, but is not limited to, polyaspartic acid, polylysine, aspartic acid, lysine and arginine, and mixtures thereof.

No special limitation on the other components of the microbial culture broth is necessary. All the components that can be used in a culture broth and that are obvious to persons skilled in the art would be suitable for use in the culture broth of the present invention. In other words, the culture broth used in the present invention can be prepared by any methods known to persons skilled in the art. For example, JP 1-174397 discloses using a culture broth composed of L-glutamic acid and peptone to grow *Bacillus subtilis* and *Bacillus natto,* which can produce γ-PGA.

In the present invention, the species of the compound used as the cross-linker is a polyglycidyl ether. For example, the polyglycidyl ether can be selected from, but is not limited to, glycerol triglycidyl either, di- or polyglycerol polyglycidyl ether and polyoxyethylene sorbitol polyglycidyl ether, and a mixture thereof.

In one embodiment of the present invention, the compound having three or more functional groups is glycerol triglycidyl ether and the compound having two functional groups is glycerol diglycidyl ether, and a mixture of them is used as the cross-linker.

The, di- or polyglycerol polyglycidyl ether can be a compound of Formula (I): 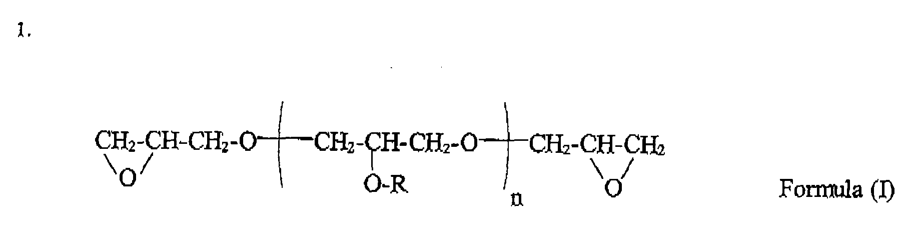 wherein R is H or 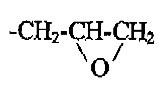 and n is from 2 to 8, preferably n is 2 to 4.

The polyoxyethylene sorbitol polyglycidyl ether can be a compound of Formula (II): 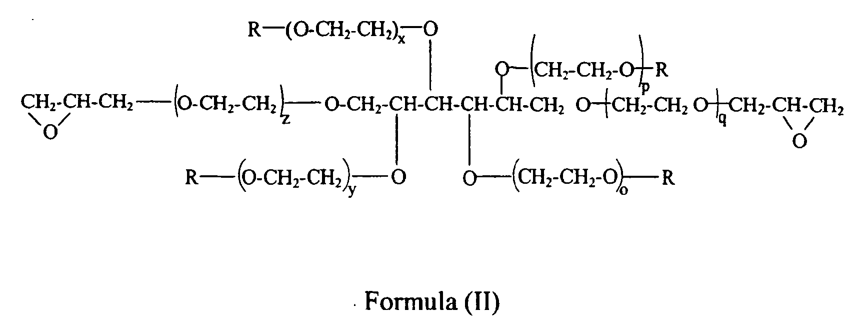 wherein R is H or 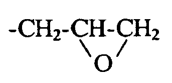 and x, y, z, o, p, and q are independently 1 to 3.

For conducting the cross-linking reaction of the present invention, the amount of the cross-linker, on the basis of the total weight of (A) and (B), is normally 0.02 to 20 wt% and preferably 0.25 to 6 wt%. If the amount of the cross-linker is below 0.02 wt%, the high water absorption rate cannot be achieved because of the insufficient cross-linking. However, if the amount of the cross-linker is greater than 20 wt%, the resulted γ-PGA hydrogels exhibit low water absorbability due to over-cross-linking.

When conducting the aforementioned cross-linking reaction, the cross-linking system is normally maintained at a pH of 3.3 to 8.5, and preferably 4.0 to 8.5. Furthermore, the reaction temperature is between 0°C and 100°C, and preferably 35°C and 85°C. Generally, it takes a longer time to complete a reaction conducted at a lower temperature, and on the contrary, a shorter time for the reaction conducted at a higher temperature. Nonetheless, if the reaction temperature is higher than 100°C, undesired side reactions, such as decomposition, will take place and influence the effectiveness of the cross-linking. In addition, carboxylic group (-COOH), carboxylate group (-COO⁻), aldehyde group (-CHO), hydroxyl (-OH), carbonyl group (-CO), sulfone group (SO₂), amino group (-NH₂) or nitro group (-NO₂), or a combination thereof to the epoxy group provided by the cross-linker is 1:1.

In the method of the present invention, the manner for carrying out the cross-linking reaction does not require any special limitation. For example, glass reactors equipped with stirrer devices or culture containers shaked in an oil or water bath can be utilized to accomplish the cross-linking reaction involved in the present invention. The method of the present invention may further comprise the steps of hydrating the cross-linked products for swelling, removing the un-cross-linked components by filtration, and drying (e.g., lyophilizing) the prepared water absorbable cross-linked product, to obtain the cross-linked product with high water absorbability.

Apparently, the method of the present invention can produce high water absorbable and stable biodegradable γ-PGA hydrogels more simply and more easily as compared with conventional methods.

The present invention further relates to a stable biodegradable, high water absorbable γ-PGA hydrogel prepared by the above method. The γ-PGA hydrogel of the present invention is effective in terms of water absorption and retention, provides up to 5,000 times water absorption rate, and can be decomposed by microbes existing in the natural environment so that its waste treatment is safer and simpler. Most importantly, since the cross-linker, which comprises a compound having three or more functional groups or a mixture of a compound having three or more functional groups and a compound having two functional groups, is used to conduct the cross-linking reaction, the γ-PGA hydrogel of the present invention has 3-dimensional inter-molecular cross-linked matrix and thus exhibits a longer stability and improved firmness and strength after full swelling in water or an aqueous medium without disintegrating or breaking down even for over 5 weeks in open atmosphere at temperature of 30°C.

The stable biodegradable, high water absorbable γ-PGA hydrogel of the present invention can be used in fields including, among others, the cosmetics fields, as moisturizers or humectants, agricultural and horticultural fields, as soil reconditioning agents, seed-coating agents, water-retaining agents for plant cultivation, immobilizing agents for manure of animals, compost adding agents, or moisturizers for feces, urine, etc.; the civil construction field, as water conditioning agents for water treatment sludge, sewage sludge, and river sewer sludge, solidifying agents, modifying agents, coagulants, or soil for reservoir; medical and hygiene fields, as absorbents for bloods or body fluids, paper diapers or tampons, or as de-odorants or controlled release drug carriers; and the bioengineering fields, as medium base for culturing microbes, plant cells or animal cells, or as immobilizing materials for bioreactors.

Moreover, since γ-PGA hydrogel of the present invention can be prepared by directly reacting the culture broth (Component (B)) with a cross-linker, it will inherently contain the components of the culture broth necessary for the growth of microbes, such as carbon source, nitrogen source, and minerals, and/or metabolites produced by microbes in the culture broth. In view of this property, the biodegradable, water absorbable γ-PGA hydrogel of the present invention is very suitable for use as an agricultural material for compost aids, seed coating agents, and desert greenification materials.

The biodegradable, water absorbable γ-PGA hydrogel of the present invention can be in any desired shapes. For example, the hydrogels can be granulated into a fixed shape or made into irregular shapes, pellets, plates, etc.

The subject invention will be further described by the following examples. Nonetheless, it should be noted that the working examples are provided for an illustration of the present invention, rather than intended to limit the scope of the present invention.

### Example 1

A 300 L culture medium containing 0.5 wt % of yeast extract, 1.5 wt % of peptone, 0.3 wt% of urea, 0.2 wt% of K₂HPO₄, 10 wt% of L-glutamic acid, and 8 wt% of glucose and having a pH of 6.8 was added to a 600 L fermentor, and then steam sterilized following the standard procedures. *Bacillus subtilis* was incubated under 37°C. After 96 hours, the culture broth contained 40 g γ-PGA per liter. Each of 15 g of the culture broth was added to 50 ml capped sample bottles into which each of 600 µl of the cross-linkers as listed in Table 1 is introduced. The reaction of the mixtures were conducted at 55°C for 20 hours in a shaker incubator, rotating at a middle speed.

Each of 1g of the reacted mixtures were taken out of the 50 ml capped sample bottles and soaked in 800 ml of water at 4°C overnight. The cross-linked hydrogel formed after hydration and swelling was then filtered through an 80-mesh metal screen and drained to dry. The weights of swollen hydrogels without obvious free water were measured and recorded. The hydrogels were re-soaked in another 800 ml of fresh water at 4°C in the same beaker overnight. The same procedure was repeated for consecutive 5 days. The cross-linked product was then tested for its water absorption rate as follows:

For the determination of water absorption rate, the cross-linked product was soaked in an excess amount of distilled water and left in the water for swelling overnight to achieve highest hydration. An 80-mesh metal screen was used to filtrate the excess amount of water to obtain the wetted cross-linked product. The wetted cross-linked product was weighed. The water absorption rate is defined as the ratio of the weight of water absorbed (the difference between the wet and dry weights) to the dry weight. The results of the water absorption rate for this example are shown in Table 1.

**Table 1**

| Cross-linker | Reaction time | Water absorption |
|---|---|---|
| | (hr) | rate |
| Polyethylene glycol diglycidyl ether | 20 | 4,500 |
| A mixture of glycerol triglycidyl ether with glycerol diglycidyl ether | 20 | 4,600 |
| Diglycerol polyglycidyl ether | 20 | 4,880 |
| Polyglycerol polyglycidyl ether | 20 | 4,950 |
| Polyoxyethylene sorbitol polyglycidyl ether | 20 | 4,750 |

polyethylene glycol diglycidyl ether is a mixture comprising compounds having the formula of 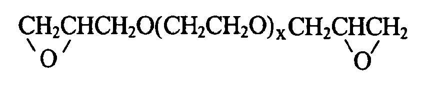 wherein x is 1, 2, 4, 9, 13 or 22

The results listed in Table 1 show that the utilization of either the compound having only two epoxy functional groups, or the compounds having three or more epoxy functional groups or a mixture of a compound having three or more epoxy functional groups and a compound having two epoxy functional groups as the cross-linker can attain at least 4,500 times water absorption rate.

### Example 2

According to the procedures illustrated in Example 1, samples of 5 wt % sodium γ-PGA solutions and a mixture of glycerol triglycidyl ether with glycerol diglycidyl ether as the cross-linker were used in another set of experiments. The pH was varied as those listed in Table 2. The reacted mixtures were further put into an culture shaker, rotating at a middle speed. The reaction was allowed to continues at 55°C for 20 hours. After the reaction was completed, the water absorption rates were determined. The results of the water absorption rates of the obtained cross-linked products are listed in Table 2.

**Table 2**

| pH | Water absorption rate |
|---|---|
| 4.0 | 435 |
| 5.0 | 610 |
| 6.0 | 3,450 |
| 8.0 | 4,550 |

### Example 3

According to the procedures illustrated in Example 1, samples of 5 wt % sodium γ-PGA solutions and diglycerol polyglycidyl ether as the cross-linker were used in another set of experiments. The pH of the solutions was adjusted to 6.5. The amounts of diglycerol polyglycidyl ether listed in Table 3 were used for the cross-linking reaction. The reaction was allowed to continue at 55°C for 20 hours. The results of water absorption rates of the obtained cross-linked products are listed in Table 3.

**Table 3**

| Amount of cross-linker (%) | Water absorption rate | | | | |
|---|---|---|---|---|---|
| | Swelling/hydration time (hr) | | | | |
| | 24 | 48 | 72 | 96 | 120 |
| 2 | 450 | 1,250 | 2,550 | 4,350 | 4,450 |
| 3 | 359 | 1,003 | 2,200 | 4,200 | 4,480 |
| 4 | - | - | 2,090 | 4,050 | 4,350 |

### Example 4

According to the procedures illustrated in Example 1, *Bacillus subtilis* was incubated. The incubation time was altered as shown in Table 4. The pH of the solutions was adjusted to 6.5. A mixture of glycerol triglycidyl ether with glycerol diglycidyl ether was used as the cross-linker. Then, the obtained culture broth was used to conduct cross-linking at 55°C for 20 hours. The results of the water absorption rates of the obtained cross-linked products are shown in Table 4.

**Table 4**

| Incubation time (hr) | Water absorption rate |
|---|---|
| 24 | a) |
| 36 | a) |
| 48 | 2,600 |
| 60 | 3,050 |
| 72 | 3,000 |
| 84 | 2,880 |
| 96 | 3,550 |

| | |
|---|---|
| a): No cross-linked hydrogel is formed. | |

### Example 5

According to the procedures illustrated in Example 1, samples of the culture broth at 96^{th} hour (3.8 wt % of γ-PGA) and samples of 3.8 wt % of γ-PGA solutions prepared from purified sodium γ-polyglutamate were used to separately react with diethylene glycol diglycidyl ether and with polyglycerol polyglycidyl ether. The pH of the solutions was adjusted to 6.5. Both of the epoxy compounds were used at 3 wt % of the culture broth or the γ-PGA solution. The cross-linking reactions were allowed to continue at 55°C for 20 hours. The resulted hydrogel samples were hydrated in excess amount of water at 4°C for 24 hours. Then, the samples were again hydrated in another fresh water. The same procedure was repeated for 3 consecutive days. The fully swollen hydrogel samples in water were kept at 30°C in open atmosphere for observing the physical stability and integrity over a period of consecutive 35 days. The soaking water of each hydrogel sample was replaced every 24 hours. The results of the physical stability and integrity of the swollen hydrogel samples were listed in Table 5.

**Table 5**

| Cross-linker (3 wt %) | Stability and integrity | | |
|---|---|---|---|
| | Condition observed after samples were placed at 30°C in open atmosphere | | |
| | 3 days | 5 days | 35 days |
| Purified γ-PGA with diethylene glycol diglycidyl ether | Partially decomposed | Completely decomposed | □ |
| Purified γ-PGA with polyglycerol polyglycidyl ether | Good and firm | Good and firm | Good and firm |
| Culture broth with diethylene glycol diglycidyl ether | Partially decomposed | Completely decomposed | □ |
| Culture broth with polyglycerol polyglycidyl ether | Good and firm | Good and firm | Good and firm |

The results in Table 5 show that the utilization of a compound having three or more epoxy functional groups, as the cross-linker can prepare a γ-PGA hydrogel with a high water adsorption rate, long stability after swelling in water, and good biodegradability.

## Claims

1. A method for the production of a biodegradable, water absorbable γ-polyglutamic acid (γ-PGA) hydrogel comprising directly cross-linking
(A) a γ-polyglutamic acid (γ-PGA), a γ-polyglutamate, or a mixture thereof, and optionally a polysaccharide containing a carboxylic and/or carboxylate group, an amino acid, or a mixture thereof; and/or
(B) a microbial culture broth containing a γ-polyglutamic acid (γ-PGA), a γ-polyglutamate, or a mixture thereof, and optionally a polysaccharide containing a carboxylic and/or carboxylate group, an amino acid, or a mixture thereof,
with a cross-linker comprising a polyglycidyl ether,
wherein each of the functional groups of the polyglycidyl ether can react with a carboxylic group (-COOH), a carboxylate group (-COO-), an aldehyde group (-CHO), a hydroxyl (-OH), a carbonyl group (-CO), a sulfone group (-SO₂), an amino group (-NH₂) or a nitro group (-NO₂), or a mixture thereof.

2. The method of claim 1, wherein the polysaccharide is a mixture of glucose, fructose, galactose, and glucuronic acid, a mixture of rhamnose, glucose, galactose, and glucuronic acid, or a polycarboxylic acid in which hyaluronic acid as the principal component.

3. The method of claim 1 or 2, wherein the amino acid is polyaspartic acid, polylysine, aspartic acid, lysine or arginine, or a mixture of any thereof.

4. The method of any one of the preceding claims, wherein the polyglycidyl ether is glycerol triglycidyl ether, di- or polyglycerol polyglycidyl ether or polyoxyethylene sorbitol polyglycidyl ether, or a mixture of any thereof.

5. The method of any one of claims 1 to 3, wherein the cross-linker comprises glycerol triglycidyl ether and the compound having two functional groups is glycerol diglycidyl ether.

6. The method of claim 4, wherein the di- or polyglycerol polyglycidyl ether is a compound of Formula (I): 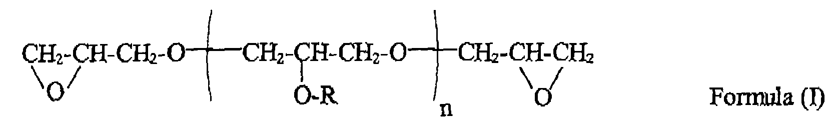 wherein R is H or 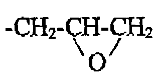 and n is from 2 to 8.

7. The method of claim 6, wherein n is from 2 to 4.

8. The method of claim 4, wherein the polyoxyethylene sorbitol polyglycidyl ether is a compound of Formula (II): 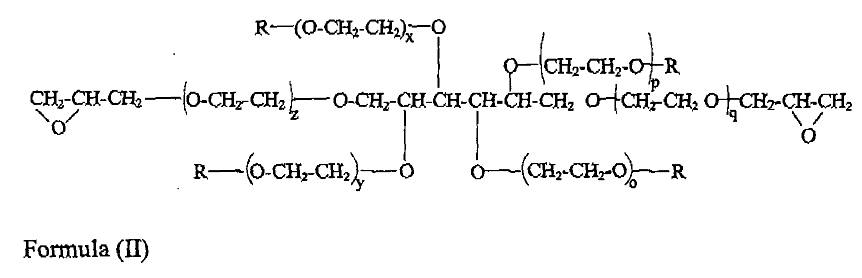 wherein R is H or 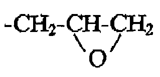 and x, y, z, o, p, and q are independently from 1 to 3.

9. The method of any one of the preceding claims, wherein the amount of the cross-linker is from 0.02 to 20 wt %, based on the total weight of (A) and (B).

10. The method of claim 9, wherein the amount of the cross-linker is from 0.25 to 6 wt %, based on the total weight of (A) and (B).

11. The method of any one of the preceding claims, wherein the cross-linking is conducted at a temperature of from 0°C to 100°C.

12. The method of claim 11, wherein the cross-linking is conducted at a temperature of from 35°C to 85°C.

13. The method of any one of the preceding claims, wherein the cross-linking is conducted at a pH of from 3.3 to 8.5.

14. The method of claim 13, wherein the cross-linking is conducted at a pH of from 4.0 to 8.5.

15. A biodegradable, water absorbable γ-polyglutamic acid (γ-PGA) hydrogel obtainable by a method according to any one of the preceding claims.

16. The hydrogel of claim 15, for use in a cosmetic, agriculture, horticulture, civil construction, a method of treatment of the human or animal body, a hygiene product, or a bioengineering application.

17. The hydrogel of claim 15 or 16 which has 3-dimensional stability after swelling in water or an aqueous medium.

18. The hydrogel of any one of claims 15 to 17 which is a long-lasting hydrogel without disintegrating or breaking down for over 5 weeks at atmospheric pressure and at a temperature of 30°C.

19. The hydrogel of any one of claims 15 to 18 comprising the components of a culture broth necessary for the growth of a microbe and/or a metabolite produced by a microbe.

20. Use of a hydrogel of any one of claims 15 to 19 in a cosmetic, agriculture, horticulture, civil construction, a hygiene product or a bioengineering application.

## Patentansprüche

1. Verfahren zur Herstellung eines biologisch abbaubaren, wasserabsorbierbaren γ-Polyglutaminsäure-Hydrogels (γ-PGA-Hydrogels), welches das direkte Vernetzen
(A) einer γ-Polyglutaminsäure (γ-PGA), eines γ-Polyglutamats oder eines Gemischs davon, und gegebenenfalls eines Polysaccharids, das eine Carbonsäure- und/oder Carboxylatgruppe enthält, einer Aminosäure oder eines Gemischs davon, und/oder
(B) einer mikrobiellen Kulturbrühe, die eine γ-Polyglutaminsäure (γ-PGA), ein γ-Polyglutamat oder ein Gemisch davon, und gegebenenfalls ein Polysaccharid, das eine Carbonsäure- und/oder Carboxylatgruppe enthält, eine Aminosäure oder ein Gemisch davon enthält,
mit einem Vernetzer umfasst, der einen Polyglycidylether umfasst,
wobei jede der funktionellen Gruppen des Polyglycidylethers mit einer Carboxylgruppe (-COOH), einer Carboxylatgruppe (-COO⁻), einer Aldehydgruppe (-CHO), einem Hydroxyl (-OH), einer Carbonylgruppe (-CO), einer Sulfongruppe (-SO₂), einer Aminogruppe (-NH₂) oder einer Nitrogruppe (-NO₂) oder einem Gemisch davon reagieren kann.

2. Verfahren nach Anspruch 1, bei dem das Polysaccharid ein Gemisch aus Glukose, Fruktose, Galaktose und Glukuronsäure, ein Gemisch aus Rhamnose, Glukose, Galaktose und Glukuronsäure, oder eine Polycarbonsäure, in der Hyaluronsäure als Hauptkomponente vorliegt, ist.

3. Verfahren nach Anspruch 1 oder 2, bei dem die Aminosäure Polyasparaginsäure, Polylysin, Asparaginsäure, Lysin oder Arginin oder ein Gemisch von jedweder bzw. jedwedem davon ist.

4. Verfahren nach einem der vorstehenden Ansprüche, bei dem der Polyglycidylether Glycerintriglycidylether, Di- oder Polyglycerinpolyglycidylether oder Polyoxyethylensorbitolpolyglycidylether oder ein Gemisch von jedwedem davon ist.

5. Verfahren nach einem der Ansprüche 1 bis 3, bei dem der Vernetzer Glycerintriglycidylether umfasst und die Verbindung mit zwei funktionellen Gruppen Glycerindiglycidylether ist.

6. Verfahren nach Anspruch 4, bei dem der Di- oder Polyglycerinpolyglycidylether eine Verbindung der Formel (I) 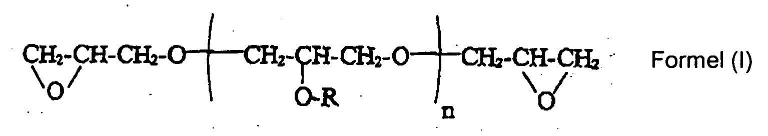 ist, worin R H oder 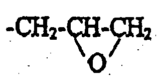 ist und n einen Wert von 2 bis 8 hat.

7. Verfahren nach Anspruch 6, bei dem n einen Wert von 2 bis 4 hat.

8. Verfahren nach Anspruch 4, bei dem der Polyoxyethylensorbitolpolyglycidylether eine Verbindung der Formel (II) 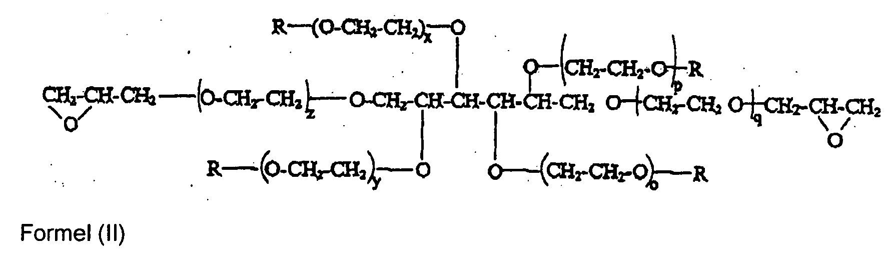 ist, worin R H oder 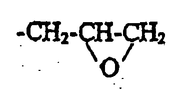 ist und x, y, z, o, p und q unabhängig einen Wert von 1 bis 3 haben.

9. Verfahren nach einem der vorstehenden Ansprüche, bei dem die Menge des Vernetzers 0,02 bis 20 Gew.-%, bezogen auf das Gesamtgewicht von (A) und (B), beträgt.

10. Verfahren nach Anspruch 9, bei dem die Menge des Vernetzers 0,25 bis 6 Gew.-%, bezogen auf das Gesamtgewicht von (A) und (B), beträgt.

11. Verfahren nach einem der vorstehenden Ansprüche, bei dem das Vernetzen bei einer Temperatur von 0°C bis 100°C durchgeführt wird.

12. Verfahren nach Anspruch 11, bei dem das Vernetzen bei einer Temperatur von 35°C bis 85°C durchgeführt wird.

13. Verfahren nach einem der vorstehenden Ansprüche, bei dem das Vernetzen bei einem pH-Wert von 3,3 bis 8,5 durchgeführt wird.

14. Verfahren nach Anspruch 13, bei dem das Vernetzen bei einem pH-Wert von 4,0 bis 8,5 durchgeführt wird.

15. Biologisch abbaubares, wasserabsorbierbares γ-Polyglutaminsäure-Hydrogel (γ-PGA-Hydrogel), das durch ein Verfahren nach einem der vorstehenden Ansprüche erhältlich ist.

16. Hydrogel nach Anspruch 15 zur Verwendung in einem Kosmetikum, in der Landwirtschaft, im Gartenbau, im Bauwesen, in einem Verfahren der Behandlung des menschlichen oder tierischen Körpers, in einem Hygieneprodukt oder in einer Biotechnologieanwendung.

17. Hydrogel nach Anspruch 15 oder 16, das nach dem Quellen in Wasser oder einem wässrigen Medium eine 3-dimensionale Stabilität aufweist.

18. Hydrogel nach einem der Ansprüche 15 bis 17, bei dem es sich um ein dauerbeständiges Hydrogel handelt, das während 5 Wochen bei Atmosphärendruck und bei einer Temperatur von 30°C sich nicht zersetzt oder nicht zerfällt.

19. Hydrogel nach einem der Ansprüche 15 bis 18, das die Komponenten einer Kulturbrühe umfasst, die für die Vermehrung einer Mikrobe und/oder eines Metaboliten, der durch eine Mikrobe erzeugt wird, erforderlich ist.

20. Verwendung eines Hydrogels nach einem der Ansprüche 15 bis 19 in einem Kosmetikum, in der Landwirtschaft, im Gartenbau, im Bauwesen, in einem Hygieneprodukt oder in einer Biotechnologieanwendung.

## Revendications

1. Procédé de préparation d'un hydrogel biodégradable, absorbant l'eau, à base d'acide γ-polyglutamique (γ-PGA), comprenant une réticulation directe de
(A) un acide γ-polyglutamique (γ-PGA), un γ-polyglutamate, ou un mélange de ceux-ci, et éventuellement un polysaccharide contenant un groupe carboxylique et/ou carboxylate, un amino-acide, ou un mélange de ceux-ci ; et/ou
(B) un bouillon de culture microbienne contenant un acide γ-polyglutamique (γ-PGA), un γ-polyglutamate, ou un mélange de ceux-ci, et éventuellement un polysaccharide contenant un groupe carboxylique et/ou carboxylate, un amino-acide, ou un mélange de ceux-ci,
avec un agent de réticulation comprenant un éther polyglycidylique,
dans lequel chacun des groupes fonctionnels de l'éther polyglycidylique peut réagir avec un groupe carboxylique (-COOH), un groupe carboxylate (-COO⁻), un groupe aldéhyde (-CHO), un groupe hydroxyle (-OH), un groupe carbonyle (-CO), un groupe sulfone (-SO₂), un groupe amino (-NH₂) ou un groupe nitro (-NO₂) ou un mélange de ceux-ci.

2. Procédé selon la revendication 1, dans lequel le polysaccharide est un mélange de glucose, de fructose, de galactose et d'acide glucuronique, un mélange de rhamnose, de glucose, de galactose et d'acide glucuronique ou un acide polycarboxylique, dans lequel l'acide hyaluronique est le principal composant.

3. Procédé selon la revendication 1 ou 2, dans lequel l'amino-acide est l'acide polyaspartique, la polylysine, l'acide aspartique, la lysine ou l'arginine ou un mélange de n'importe lesquels de ceux-ci.

4. Procédé selon l'une quelconque des revendications précédentes, dans lequel l'éther polyglycidylique est l'éther triglycidylique de glycérol, l'éther polyglycidylique de di- ou de polyglycérol ou l'éther polyglycidylique de polyoxyéthylènesorbitol ou un mélange de n'importe lesquels de ceux-ci.

5. Procédé selon l'une quelconque des revendications 1 à 3, dans lequel l'agent de réticulation comprend l'éther triglycidylique de glycérol, et le composé ayant deux groupes fonctionnels est l'éther diglycidylique de glycérol.

6. Procédé selon la revendication 4, dans lequel l'éther polyglycidylique de di- ou polyglycérol est un composé de formule (I) : 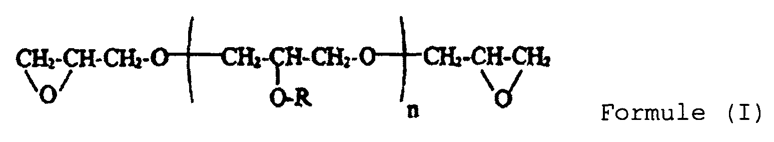 dans laquelle R est H ou 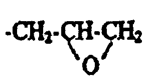 et n vaut de 2 à 8.

7. Procédé selon la revendication 6, dans lequel n vaut de 2 à 4.

8. Procédé selon la revendication 4, dans lequel l'éther polyglycidylique de polyoxyéthylènesorbitol est un composé de formule (II) : 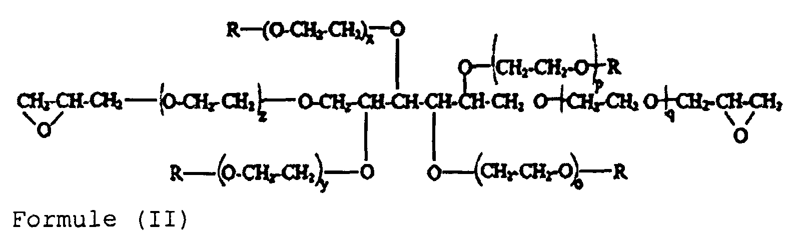 dans laquelle R est H ou 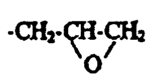 et x, y, z, o, p et q valent indépendamment de 1 à 3.

9. Procédé selon l'une quelconque des revendications précédentes, dans lequel la quantité de l'agent de réticulation est de 0,02 à 20 % en poids par rapport au poids total de (A) et (B).

10. Procédé selon la revendication 9, dans lequel la quantité de l'agent de réticulation est de 0,25 à 6 % en poids par rapport au poids total de (A) et (B).

11. Procédé selon l'une quelconque des revendications précédentes, dans lequel on effectue la réticulation à une température de 0°C à 100°C.

12. Procédé selon la revendication 11, dans lequel on effectue la réticulation à une température de 35°C à 85°C.

13. Procédé selon l'une quelconque des revendications précédentes, dans lequel on effectue la réticulation à un pH de 3,3 à 8,5.

14. Procédé selon la revendication 13, dans lequel on effectue la réticulation à un pH de 4,0 à 8,5.

15. Hydrogel biodégradable absorbant l'eau à base d'acide γ-polyglutamique (γ-PGA) pouvant être obtenu au moyen d'un procédé selon l'une quelconque des revendications précédentes.

16. Hydrogel selon la revendication 15 pour une utilisation dans un produit cosmétique, en agriculture, en horticulture, dans une construction civile, un procédé de traitement du corps humain ou animal, un produit hygiénique ou une application en bio-ingénierie.

17. Hydrogel selon la revendication 15 ou 16, qui a une stabilité tridimensionnelle après un gonflement dans de l'eau ou dans un milieu aqueux.

18. Hydrogel selon l'une quelconque des revendications 15 à 17, qui est un hydrogel de longue durée sans désintégration ou décomposition pendant plus de 5 semaines à la pression atmosphérique et à une température de 30°C.

19. Hydrogel selon l'une quelconque des revendications 15 à 18, comprenant les composants d'un bouillon de culture nécessaire pour la croissance d'un microbe et/ou d'un métabolite produit par un microbe.

20. Utilisation d'un hydrogel selon l'une quelconque des revendications 15 à 19 dans un produit cosmétique, en agriculture, en horticulture, dans une construction civile, un produit hygiénique ou une application en bio-ingénierie.
